# EUROPEAN PATENT SPECIFICATION

(11) **EP 1 404 237 B1**
(45) Date of publication and mention of the grant of the patent: **12.09.2007**
(21) Application number: 01945597.1
(22) Date of filing: 28.06.2001
(51) Int. Cl.: A61B 17/22

(54) **FOREIGN BODY RETRIEVAL DEVICE**
VORRICHTUNG ZUM EINFANGEN VON FREMDKÖRPERN
DISPOSITIF DE RECUPERATION DE CORPS ETRANGER

(43) Date of publication of application: 07.04.2004
(73) Proprietor: Lithotech Medical Ltd, 12900 Katzrin (IL)
(72) Inventor: KHACHIN, Vladimir, 634004 Tomsk (RU); KHACHIN, Stepan, 634004 Tomsk (RU)
(74) Representative: Casey, Lindsay Joseph
(86) International application number: PCT/IL2001/000591
(87) International publication number: WO 2003/002006

(56) References cited:
- WO-A-01/10290
- WO-A-92/16153
- WO-A-96/23446
- US-A- 6 093 196
- DATABASE WPI Section PQ, Derwent Publications Ltd., London, GB; Class P31, AN 1995-176630 XP002172655 & SU 2 022 528 A (VOZIANOV), 15 November 1994 (1994-11-15) cited in the application

## Description

### Field of the invention

The present invention relates to a surgical apparatus for immobilization and evacuation foreign objects preferably from a human body. In particular the present invention refers to surgical extractors suitable for use in urological treatments like extracting calculi appearing in the biliary or urinary system.

It should be understood however that the present invention is not limited to the urological treatment of a human body. It can be successfully employed for surgical treatments of animals as well. Furthermore the present invention is not limited strictly to extracting calculi during the urological treatment. It is suitable for other surgical treatments, which might require retrieval of foreign objects from the body systems, e.g. from blood vessels etc.

### Background of the invention

Despite the fact that by virtue of available modern video-assisted endoscopic instrumentation significant progress has been achieved in urological less invasive treatment in general and in the extracting of foreign objects in particular, nevertheless the evacuation of such objects like calculi from the body remains a challenge for a surgeon.

As an example of complicated and time-consuming surgical treatment one can mention removal of calculi of different size and characteristics from various sites along the urinary tract and from various locations within the body, e.g. removal of gallstones and kidney stones. This challenge results in development of a variety of surgical tools for stone retrieval without the need for major surgery. The calculi retrieval tools, or so-called surgical extractors usually comprise a flexible tubular catheter formed as a tubular sheath adapted to penetrate along the body passages to reach the location where the object to be evacuated resides. Within the catheter a wire or cable is located, which can be manipulated from the outside at the catheter's proximal end. The cable is connected with a basket-like element deployed within the sheet at the catheter's distal end. The basket like element consists of flexible wires made either of stainless steel or a memory shape material or any other material capable to provide the basket with elasticity. Depending on the manipulation the basket may either to retract inside the sheet to allow penetration of the catheter via passage or to protract from the catheter. In protracted position the wires open due to the elasticity of their material and form a cage thus to allow entering the object inside the basket through the open spaces left between its adjacent wires. Further retraction of the basket inside the sheath results in collapsing the cage and imprisonment the object in the basket. Removal of the catheter will enable the whole to be removed from the body organ together with the object immobilized within the basket. An example of the above-described surgical extractor is disclosed in FR 1197808.

It can be easily appreciated that the particular design of the basket-like element is crucial for the easy immobilization and reliable retaining the object during evacuation.

Therefore various attempts to devise such basket-like element are known in the art, for example in http://www.bsci.com. According to recent classification suggested by Boston Scientific Corporation the currently employed basket-like elements for stone retrieval can be broadly categorized into following groups:
1. Flatwire baskets
2. Helical baskets
3. Multi-wire baskets

The reason for the above situation lies in the fact that there is still felt a strong need in a simple, inexpensive and convenient surgical tool suitable for reliable and efficient evacuation of foreign objects from the body irrespective of the size of the object and its location within the body.

An example of a flatwire basket is disclosed in US6183482. This basket comprises one or more legs to retrieve the calculi. At least one of the legs has an inner and outer surface, which is curved to render the basket atraumatic. The inner surface can be flat such that the leg has a D-shaped cross-sectional configuration. It is reported in the literature that flatwire baskets minimize lateral basket wires movement and this facilitates and improves stone capture. Furthermore flatwire baskets open reliably even in an undilated ureter. Flatwire baskets are available in a wide range of sizes and wire configurations. A method of manufacturing of such baskets is described in US 5792145.

Unfortunately the intrinsic disadvantage of flatwire baskets is their unsuitability for capturing and immobilization of relatively small calculi, since the amount of wires in such baskets is limited to very few and the size of open space between the adjacent wires remains too large.

In an attempt to solve this problem and to allow capturing and retaining of small calculi so-called helical baskets were devised. Examples surgical extractors equipped with helical baskets can be found in US3496330, US 6190394, US 4347846.

It is stated in the literature, that helical baskets incorporate strong, flexible wire construction in a spiraled shape design. The streamlined, spiraled shape is well suited for efficient, effective stone capture. As the basket is drawn back over the entrapped calculi, the configuration of the wires "sweeps" the stone into the basket.

In US 5496330 is disclosed helical basket formed as a plurality of strands encased in a sheath and wrapped in a helical form. Displacement of a portion of the threads from the sheath causes their wide angularly disposition to form a basket for retrieving the object. The threads comprise a plurality of individual filaments that are closely spaced through the length of the basket. By virtue of strands, formed as multiple, closely situated adjacent wires the number of contact points with entrapped calculi is increased without requiring a concomitant increase in the size of the overlying sheath or in the difficulty placing the basket over the calculi.

Nevertheless, one can assume that the above-mentioned advantage associated with the increased number of contact points may limit this basket to treatment of small calculi and render this basket less suitable for immobilization of relatively large calculi. The reason for this is close angular disposition of the filaments that prevents easy entering of large calculi in the limited open space between the adjacent filaments.

In US 6190394 is disclosed medical retrieval basket formed as plurality of flexible elements that are outwardly disposed to form a cage for entrapping objects therein.

This basket was devised to enable efficient entrapment of the objects and their reliable holding within the cage. To achieve this goal the basket is manipulated by rotation and employs flexible elements having different sizes, different cross-sectional configuration and/or different spacing. In one embodiment the cage consists of non-twisted flexible elements as in flatwire basket design. In the other embodiment the flexible elements are helically twisted. In both embodiments the cage is symmetrical with respect to a plane drawn perpendicularly to the middle of the cage length. It can be assumed however that the above-mentioned intrinsic disadvantages associated with flatwire and helical baskets would be characteristic also to the basket disclosed in this patent. The further disadvantage of the helical design is associated with the fact that parallel-directed helically shaped filaments are prone to entanglement.

In US 4347846 is described surgical extractor employing a cage or basket formed by steel wires and disposed in a helical path. Some of the wires follow a helix in clockwise direction, while other wires, in equal number follow a helix in anticlockwise direction.

By virtue of this extractor the reliability of retention of the body during evacuation is improved, since the body may enter within the extractor sheath when the cage is being retracted. This prevents accidental escape of the body from the cage. Furthermore, by virtue of opposite direction of helically shaped filaments any danger of their twisting and entanglement of the cage is avoided.

It should be emphasized that the general shape of the cage employed in this extractor is very similar to the shape of helical basket disclosed in US 6190394. This shape is also symmetrical to a plane, drawn perpendicularly to the middle of the basket's length. Therefore the same above-mentioned intrinsic disadvantages are characteristic to this helical basket as well. Furthermore, since the filaments are not secured together and only have points of passive contact in which they overlap one can assume that the cage will neither have sufficient radial rigidity to prevent escape of the calculi between adjacent filaments, nor sufficient dilatation ability to provide enough room between the entrapped calculi and the tissue of the passage. Thus either loss of calculi during evacuation or damage of the adjacent tissue might occur during the treatment.

In an attempt to overcome the disadvantages associated with entrapment and retention calculi of various sizes a multi-wire parachute basket design was devised.

Two sections define this basket. In the first section the amount of filaments is small and they are in spaced relationship to enable easy passage of the calculi through the spaces between the filaments. The second section is formed as plurality of filaments, extending from the filaments of the first section and defining a multi-wire cage.

Since in this cage the filaments are in close relationship it is possible to ensure more complete engagement of the captured calculi when the basket in protracted position and its more reliable holding when the basket is being retracted within the sheath.

One early attempt to devise a basket in accordance with the parachute concept is described in US 3472230. In this patent is disclosed a retrieval basket made of four spring wires connected to a slide. An umbrella made of suitable flexible web material is connected to the distal ends of the wires so that upon deployment of the basket the edges of the umbrella form a scraper. Retracting the basket is associated with scraping the debris from a body passage and its retaining within the umbrella.

The other example of retrieval parachute basket, provided with web umbrella is presented in US 4790812.

The disadvantage of parachute baskets provided with web umbrella is associated with difficulty in manufacturing and with relatively large size, which the web umbrella requires in the compacted condition.

The further progress in parachute baskets was achieved by devising multi-wire parachute baskets. The early attempt to design such baskets is described for example in SU 1036325. The more recent examples are disclosed in SU 202528 and US 6168603.

Surgical extractor described in SU 1036325 is provided with multi-wire parachute basket formed with a first section, which is proximal to the sheath and with a second section, which is distal thereto. The proximal section comprises two separate branches extending from the sheath in a V-shaped fashion. The branches comprise strings that are woven from the individual wire filaments. From distal ends of the branches emerge individual wire filaments, which are bent as elongated elliptical loops. The loops emerging from the branches meet in a common point at the distal end of the extractor. Longitudinally extending within the loops additional wire filaments are provided. These filaments divide the loops into supplemental branches, which together define the second section of the basket.

The disadvantage of this basket is associated with the fact it still does not always ensures reliable retention of the calculi, since amount of branches along the basket is invariant and their filaments are parallel and separate. Thus small calculi might escape through the room between the branches of the second section.

On the other hand, the elongated elliptically configured separate branches do not prevent possible damage to the nearby tissue caused by the large calculi that might protrude outside.

Substantially similar construction of multi-wire parachute basket is disclosed in US6168603. In this US patent is described surgical extractor at which distal end is arranged a plurality of wires defining a retrieval basket. Each wire comprises a first portion having an individual strand and a second portion comprising a plurality of filaments. When a retaining sheath of the extractor is retracted the wires formed of a shape memory material expand. Each strand assumes a spaced relationship to define a first section of the basket. Plurality of filaments defines a second section of the basket. Widely spaced wires of the first section promote capturing the object and closely spaced wires of the second section enable retention of the captured object. It is worth to mention that in one of the embodiments the second section is formed from filaments, which are helically wound and angularly spaced.

In SU 202258 is disclosed surgical extractor provided with a basket formed from at least two branches made of metallic strings. The main branches define the first section of the basket, which is intended for entering the calculus within the basket.

Each branch forms at its distal end sub branches, which are made of two main sub branch and one auxiliary sub branch. The sub branches define the second section of the basket. The sub branches and the auxiliary branches have different thickness and different elasticity. By virtue of this basket the reliability of entrapment and immobilization of the entrapped calculus is improved, since its construction allows variation of the amount and arrangement of the sub branches.

Unfortunately the above mentioned multi-wire parachute baskets only partially solve the problem of reliable capturing, immobilization and holding of a calculi irrespective of their size. The reason for this is the size of the open space along the branches, which is equal to the length of filaments of the second section. Calculi, which size is shorter than this length can easily escape from the basket. Furthermore, in the above baskets the filaments of neighboring branches are either fully separate (as in SU 1036325) or only passively overlap (as in US 6168603, SU 2022528) and they are not entwined. By virtue of this provision the radial rigidity of the cage is very limited and might be not sufficient to prevent possibility that the adjacent filaments will be spread apart by the calculus during its entrapment and thus the calculus can escape. Insufficient radial rigidity is associated also with the possibility for damaging adjacent body tissues by the entrapped calculus protruding through open space formed by the spread filaments.

The insufficient radial rigidity deteriorates dilatation ability of the whole basket and therefore contributes to the possibility for damage to the nearby body tissues.

In conclusion it should be emphasized that despite the fact that numerous surgical extractors employing retrieval baskets for evacuation of calculi or other objects from the body have been devised there is still a need for a new and improved device that will ensure efficient, reliable, easy and safe surgical treatment of foreign objects.

### Object of the invention

According to an aspect of the present invention, there is provided a retrieval basket as specified in claim 1.

The main object of the present invention is to provide a new and improved surgical tool enabling sufficiently reduce or overcome the above-mentioned drawbacks of the known in the art tools.

In particular the first object of the invention is to provide a new and improved surgical tool, ensuring reliable immobilization and removal of an object from the body lumen irrespective of the object's size and configuration.

Still further object of the invention is to provide a new and improved surgical tool, which is provided with retrieval basket defined by increased structural rigidity and dilatation ability and thus reducing probability for traumatizing of adjacent body tissues.

Another object of the invention is to provide improved surgical tool, defined by easy and fast protracting and retracting from the sheath and thus enabling more reliable functioning during the surgical treatment.

Yet another object of the invention is to provide new and inexpensive surgical tool, ensuring adaptation to elastic properties of the surrounding body tissues.

The above and other objects and advantages of the present invention can be achieved in accordance with the following combination of its essential features, referring to different embodiments thereof as a surgical apparatus and as a retrieval basket.

According to the embodiment of the invention, which refers to an apparatus it comprises a retrieval basket and a basket control means. The basket is suitable for immobilization the object and retaining thereof during evacuation and is defined by a first section, suitable for capturing the object and by a second section, suitable for retention the captured object. The first section comprises at least two branches, wherein distal ends of at least some of said branches are provided with loops emerging from the distal ends, said loops are configured from individual wire filaments to collectively define the second section of the basket. The basket control means is suitable for bringing the basket to the object and is adapted for manipulating the basket. The basket control means comprises a tubular sheath member to be inserted within the body. The basket control means is suitable either for retracting the basket within the sheath to enable bringing the sheath within the body or for protracting the basket from the sheath to enable opening of the basket.

In accordance with the embodiment of the invention, referring to a basket it is defined by a first section, suitable for capturing the object and by a second section, suitable for retention the captured object. The first section comprises at least two branches, wherein distal ends of at least some of said branches are provided with loops emerging from the distal ends, said loops are configured from individual wire filaments to collectively define the second section of the basket, wherein distal ends of at least some of the loops of the second section are in overlapping relationship to form dense meshed structure imparting structural rigidity and dilatation ability to the second section of the basket when it is opened.

The present invention in its various embodiments has only been summarized briefly. For better understanding of the present invention as well of its advantages, reference will now be made to the following description of its embodiments with reference to the accompanying drawings.

### Brief description of the drawings

Fig.1 shows general view of the apparatus of the invention
Figs.2a-2d depict various views of the basket provided with two branches
Figs. 3a-3d depict various views of the basket provided with three branches
Figs.4a-4d are various views of the basket provided with three branches, each of which is configured with two loops
Figs.5a-5d are various views of the basket provided with four branches, each of which is equipped with one loop
Figs. 6a-6d are various views of the basket shown in Figs. 5a-5d in which the loops are configured in an 8-like fashion
Figs. 7a-7f show schematically how distal ends of the loops are intertwined

### Detailed description of specific embodiments

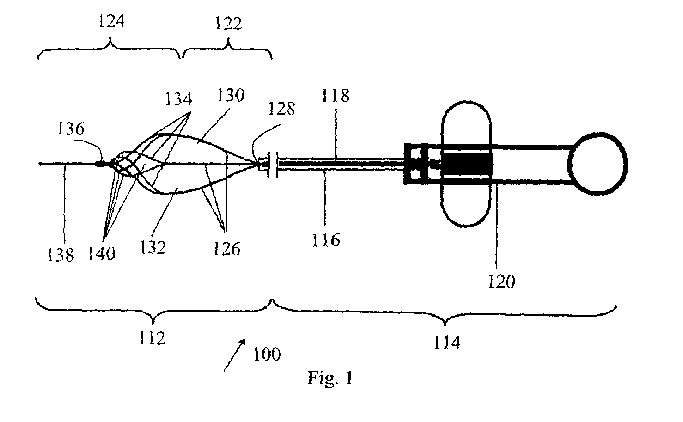

Referring now to Fig.1 it is shown general view of a surgical apparatus 100, which is devised for retrieval of various calculi or other objects from human's or animal's body during urological treatment of biliary or urinary systems.

This treatment may include evacuation of stones from urethra, evacuation of gall stones, kidney stones etc. The apparatus of the invention can be used also in other surgical treatments in combination with other surgical instruments and equipment, e.g. for destruction of calculi, etc. The apparatus of the invention comprises a retrieval basket portion 112 and a control means portion 114. The retrieval basket portion is suitable both for immobilization the object to be evacuated and for retaining thereof during the evacuation. The control means portion is suitable for manipulating the retrieval portion. For the sake of brevity the retrieval basket portion will be referred to further as retrieval basket and the control means portion as control means. Construction of the surgical apparatus of the present invention is similar to already mentioned surgical extractors. It includes flexible tubular catheter and a retrieval basket. The catheter is configured as a sheath 116 adapted to penetrate along the body passages near the location of the object. The basket is connected to a pushing rod 118 or cable or wire, which is arranged within the catheter and is connected to a manipulator 120. A surgeon by virtue of manipulator can manipulate the pushing rod and thus the basket either can be retracted within the catheter or protracted therefrom. The surgeon by holding the manipulator can also to maneuver the catheter within the body organ, e.g. to displace it by turning, pushing or pulling. In practice the surgeon brings catheter behind the object to be evacuated and then protracts the basket from the catheter. Once the basket is protracted it opens due to resiliency and is ready for receiving the object to be entrapped thereinto. The surgeon pulls catheter together with the basket until the basket is put on the object and thus evacuating the entrapped object from the body organ can be initiated.

The retrieval basket comprises a first section 122, which is proximal the control means and a second section 124, which is distal thereto. The first section is made of strands 126 arranged in branches, which meet in a meeting point 128, where the retrieval basket is connected to the pushing rod of the control means. The strands consist of wire filaments, which are entwined and spatially arranged in such manners that upon protracting from the sheath they readily spread out. The filaments may have diameter 0.1-0.4 mm. The branches define the first section of the basket. In the open condition the first section of the basket has large open spaces, 130,132 left between the adjacent branches. It can be easily appreciated that by virtue of open spaces the immobilization of the object and receiving thereof inside the fist section becomes easy and convenient during the first stage of the treatment when the surgeon starts pulling catheter with the basket put on the object.

As seen in Fig.1 the strands are arranged in three branches and define the first section or the basket. In practice it might be sufficient if only two branches define the first section.

From the distal ends of the branches emerge loops 134, which are configured from individual wire filaments. The loops are configured from the same wire filaments from which the branches are entwined. It is possible however that the loops are made of separate wire filaments and connected to the respective branches, e.g. by soldering. The most distal ends of the loops are secured together by a tip 136. Behind the tip the wire filaments are intertwined to form a straight short section 138 of the basket. This section functions as a guide to facilitate displacement of the basket within the sheath and to facilitate penetration and movement of whole device within the body organs.

In accordance with the invention the adjacent to the tip parts of loops are spatially arranged in such a manner that at least some of them overlap. The overlapping wire filaments intersect each other and define collectively the second section of the basket.

Furthermore, in accordance with the invention distal ends of at least some of the loops also intertwine one with another. By virtue of this arrangement the second section of the basket is configured as a spatial net, having cell structure, consisting of relatively small cells 140, suitable to retain therein reliably entrapped objects. Naturally, the size of the cells 140 is decreased towards the distal end.

It has been empirically revealed that this cell structure has improved radial rigidity dilatation ability thus rendering the whole basket less traumatic.

The retention ability of the basket depends on the size and shape of the cells, which in turn could be controlled by the size of loops, their amount and spatial arrangement of their distal ends. These features of the invention will be described below with reference to further drawings. Furthermore, although it is not shown specifically in the drawings it should be understood that in accordance with the invention the amount of loops emerging from the branches could be similar or dissimilar and so their size.

The branches and loops are configured from wire filaments made of metallic or non-metallic material having sufficient elasticity to enable opening the basket when it is protracted from the sheath. This material should be also biologically inert. In practice the wire filaments could be made of metallic material, e.g. stainless steel or organic material, e.g. Capron. It is also recommendable to coat metallic wire filaments by an inert coating, e.g. Teflon.

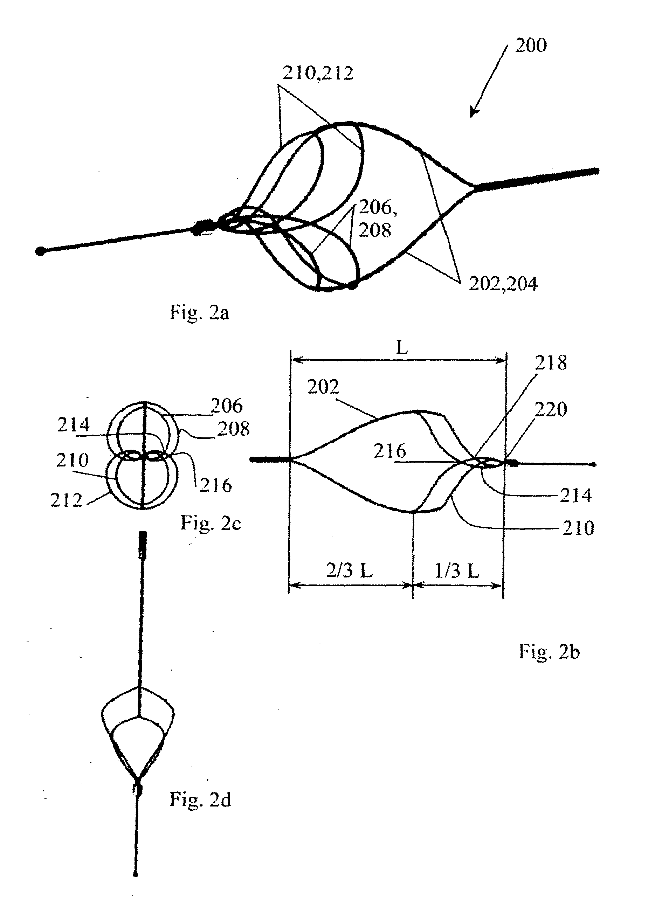

Now with reference to Figs.2a-c it will be described in more details the configuration of retrieval basket in accordance with an embodiment of the invention. In this embodiment, which in general is similar to the embodiment shown in Fig.1 only two branches 202,204 define the first section of the basket 200 and thus relatively large open space is available between the branches enabling easy immobilization of large calculi. Branch 202 is provided with a couple of loops 206,208 and branch 204 is provided with respective couple of loops 210,212 having dissimilar size. It is seen that loop 206 is less than loop 208 and loop 210 is less than loop 212. Distal ends of the loops overlap and intersect in several points. For the sake of simplicity only two points, namely 214 and 216 are designated in Fig.2b to show intersection of the wires, from which loops 210 and 206 are configured. It can be seen that by virtue of this spatial arrangement net-like structure is formed, consisting of small cells, two of which, are designated in Fig.2b by numerals 218, 220. It should be appreciated that by virtue of loops having dissimilar size it is possible to form additional cells without introducing new branches.

It is not shown specifically by should be understood that wire filaments, from which the loops are made can also intertwine one with another thus submitting additional structural rigidity to the net-like structure. It can be appreciated that by virtue of this configuration very reliable retention of the entrapped calculi or object is ensured when it is evacuated from the body organ.

Thus, in this embodiment the retrieval basket consists of a first section, defined by branches 202,204, and of a second section, defined by loops 206 208,210,212 and small cells. In practice it is advantageous if the length of the first section is about 2/3 of the total basket length and the length of the second section is about 1/3 of the total length. This is schematically shown in Fig.2a, the numeral L designates the total length.

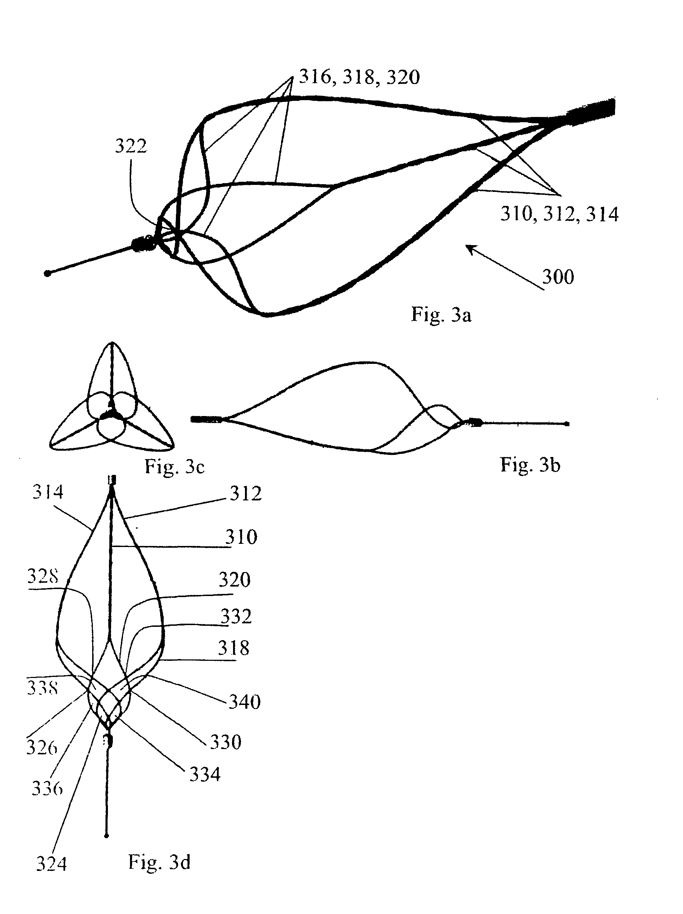

Still further embodiment of the invention is presented in Figs.3a-3d. Here three similar branches 310,312,314 define first section of basket 300. Each branch is provided with a dedicated loop 316,318, 320, emerging from the distal end of the corresponding branch. Distal ends of the loops spatially overlap and intersect. As best seen in Fig.3d, loops 316 and 318 intersect in points 322 and 324, loops 320 and 316 intersect in points 326,328 and loops 320,318 intersect in points 330,332. By virtue of this provision net-like structure is formed, consisting of small cells, some of which are designated in Fig.3d by numerals 334,336,335,340. The loops and cells collectively define second section of basket 300, in which the entrapped object can be reliably retained during evacuation. Similarly to the previous embodiment at least some of the individual wire filaments, from which the loops are made can also intertwine one with another and thus a net-like structure is formed, having improved structural rigidity.

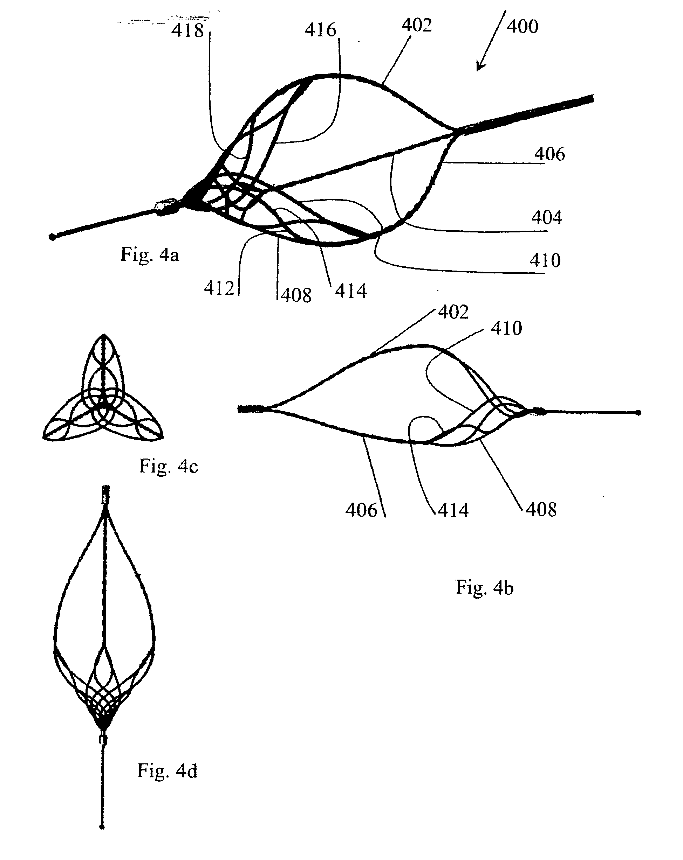

Referring now to Figs. 4a-4c another embodiment of the retrieval basket will be explained. In this embodiment retrieval basket 400 consists of three branches 402,404,406. Each branch is made of four entwined wire filaments. At the distal end of each branch the filaments are arranged to form two loops, one of which has regular shape, while the second one is twisted and has an 8-shape configuration.

It is seen, for example, in Figs.4a and 4b that wires 408, 410,412, 414 emerge from branch 406. Two wire filaments 408 and 410 form regular loop, while two other wire filaments 412 and 414 are twisted and form an 8-shaped loop.

Distal ends of the loops are in overlapping relationship and their wire filaments intersect. Some of the wire filaments are also intertwined. It is seen, for example in Fig.4a, that wire filament 410 of the loop referring to branch 406 intersects with wire filaments 416,418 of two loops emerging from branch 402. At the same time wire filament 416 of the loop referring to branch 402 intertwines with wire filaments 410 and 412 of the loop emerging from branch 406. It can be readily appreciated that by virtue of this arrangement the whole second section of the basket becomes a net, having dense meshed structure since twisted loops also contribute to formation of cells. The cells formed by intersection of the distal ends and the cells formed due to twisting collectively define the net-shape structure, which is especially suitable for reliable retention of small objects or calculi. At the same time sufficient space is still reserved between the branches to allow easy entrapment of relatively large objects or calculi.

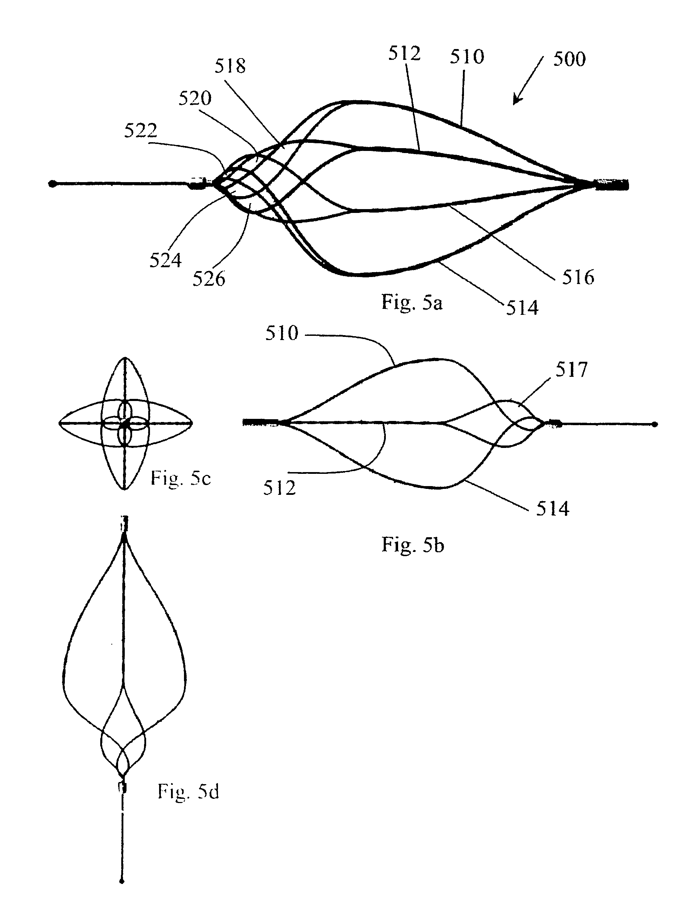

Now with reference to Figs.5a-5d another embodiment of the invention will be explained. As best seen in Fig.5a basket 500 has its first section, defined by four branches 510,512,514,516. Each branch is provided at the distal end thereof with a loop, having elongated shape. One such loop emerging from branch 512 is designated in Fig.5b by numeral 517. Distal ends of the loops are in overlapping relationship and their wire filaments intersect with formation of plurality of small cells. Some of the cells are designated by numerals 515,520,522,524, 526,528. The cells together with loops define spatially the second section of the basket. It is not seen in the drawings, but should be understood that similarly to the previous embodiment at least some of the filaments, from which the loops are formed, can also intertwine, thus rendering the resulting structure additional structural rigidity.

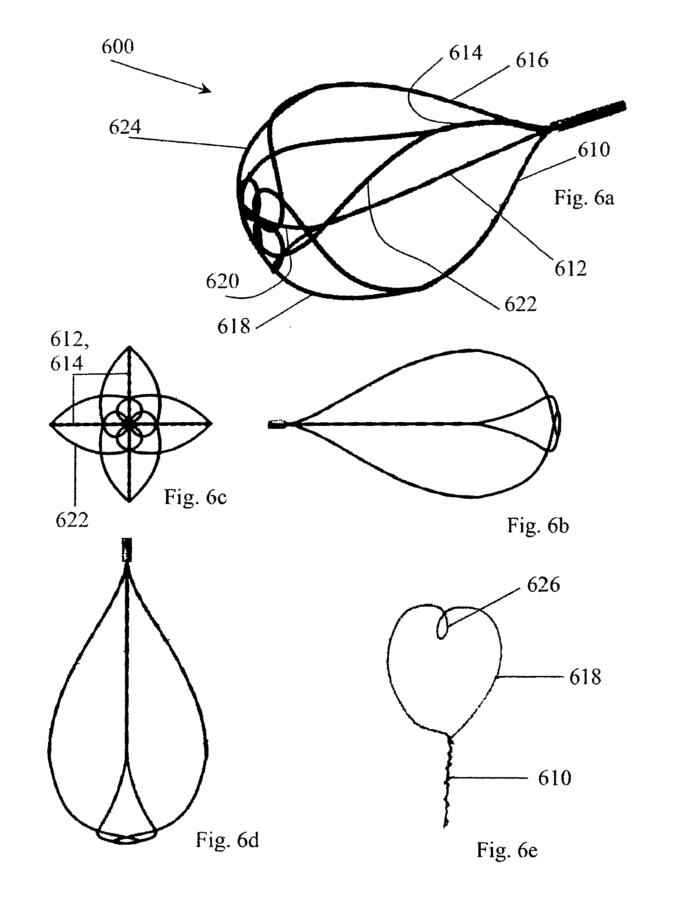

Referring to Figs.6a-6e it is shown still further embodiment of the invention. In accordance with the embodiment four branches 610,612,614,616, which are provided at the distal ends thereof with corresponding loops 618,620,622,624, form basket 600. In contrast to the previous embodiments the loops are provided with dissimilar shape. The loops are bent in such a manner that additional small loops are formed at the distal end of each loop. In Fig.6e is shown one such loop 618, emerging from branch 610 and terminating by additional small loop 626. The additional loops are provided with circular shape and they intersect and intertwine thus defining dense net-like structure of the second section of the basket.

It can be readily appreciated that by providing the loops with various shape it is possible to vary also the size and shape of the cells, formed at intersection of the loops and thus so to control the retention ability of the basket. In practice the size of cells is 2-10 mm and they allow efficient entrapment of calculi with the size of more than 2 mm.

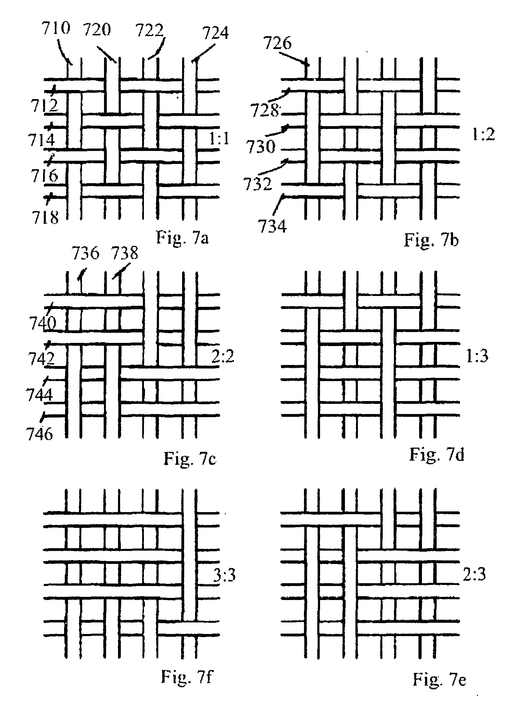

Referring now to Figs.7a-7f it will be explained how individual wire filaments, from which the loops are made, could intersect and/or intertwine. By intersecting here is meant such arrangement of the filaments, in which one element passes through the perpendicularly directed filament and they overlap, i.e. one of the filaments is always over or under the other filament. By intertwining here is meant the situation when at least one filament twines with the perpendicularly directed filaments, i.e. it goes first above and then under them.

In Figs.7a-7f is shown schematically and with exaggeration different patterns corresponding to possible arrangement of individual filaments defining the cells. For the sake of simplicity the wire filaments are depicted as vertical and horizontal bands intersecting at right angle and defining orthogonal pattern, consisting of four vertical and four horizontal bands. It should be understood however that in reality thin wire filaments form the cells. The filaments are directed with respect to each other not necessarily at right angle and their amount is not limited to four by four pattern.

In Fig.7a is shown a pattern in which all wire filaments intertwine one with another, i.e. each horizontal wire filament intertwines with all vertical filaments and vice versa. It is seen, for example, that vertical filament 710 goes first under horizontal filament 712, then above horizontal filament 714, then again under horizontal filament 716 and finally again above horizontal filament 718. On the other hand horizontal filament 712 goes first above vertical filament 710, then under vertical filament 720, then again above vertical filament 722 and then again under vertical filament 724. The rest of filaments are arranged similarly.

In Fig.7b is depicted another situation, in which the filaments both intertwine and overlap with intersection. It is seen that vertical filament 726 intertwines with horizontal filaments 725,730,734 and intersects with the perpendicularly directed filament 732.

In Fig.7c is shown still new pattern, consisting of intertwined and intersecting filaments. It is seen for example that two neighboring vertical filaments 736,738 go under two neighboring horizontal filaments 740,742 and then above two next horizontal filaments 744,746.

In Figs.7d-7f are shown further possible patterns, consisting of intertwining and intersecting filaments.

In the last pattern, seen in Fig.7f each filament intersects with three perpendicular filaments and intertwines with only one filament.

It can be realized that the patterns depicted in Figs.7a and 7f present two extreme situations, corresponding respectively to the pattern in which all filaments intertwine and to the pattern in which only one filament intertwines, while the other filaments intersect.

Having explained various cells patterns, in which the filaments of the loops might be arranged still another embodiment of the apparatus of the invention will be disclosed. In this embodiment opening of the basket is ensured not by elasticity of the wires material as in the previous embodiments, but by virtue of thermo mechanical shape memory characteristic of the material from which the wires are made. The suitable alloy may be super elastic alloy based on Ni and Ti, like Nitinol or any other suitable commercially available alloy having shape memory ability, i.e. the ability to return to some previously defined shape or size when subjected to the appropriate thermal procedure. In this embodiment the basket control means comprises power supply source for passing electric current through the wires and capable to cause them to change their shape due to heating. Insulating coating, which can be made of Teflon, coats the wires. The advantage of Teflon is its thermal resistance and low coefficient of mechanical friction, which leads to additional reduction of traumatism.

Furthermore, manufacturing of the basket wires from Nitinol renders the whole basket ability to undergo reversible deformation up to 10%, which is close to the dilatation ability of living tissues. It can be readily appreciated that basket made of Nitinol becomes compatible both biologically and mechanically with the adjacent tissues, since it is capable better to copy the topography of the organ in which the basket resides. By virtue of improved compatibility the basket becomes less traumatic. Still further advantage of t using Nitinol is associated with the "superelastic" properties of this material and the capability of this alloy to improve its "rigidity" when the temperature increases. Introducing of a catheter with deployed therein Nitinol basket into a body organ, having temperature higher then the ambient temperature increases elasticity of the basket and its dilatation ability, which improves the ability of the basket to reliably entrap and retain the object or calculi to be retrieved. It should be also mentioned, that due to the "superelastic" properties of

Nitinol surgical extractors employing baskets made of it have longer service life, then extractors with baskets made of stainless steel.

It can be appreciated that by virtue of the above-described construction of the retrieval basket, provided with overlapping loops, the whole surgical apparatus of the present invention becomes very simple, inexpensive, easy and reliable in operation. The surgical tool of the invention is capable to conveniently immobilize and reliably retain both small and large objects and at the same time it is less traumatic due to basket's improved structural rigidity and dilatation ability.

It should be understood that the present invention should not be limited to the above described example and embodiments. One ordinarily skilled in the art can make changes and modifications without deviation from the scope of the invention.

It should be appreciated that the features disclosed in the foregoing description, and/or in the following claims, and/or in the accompanying drawings may, both separately and in any combination thereof, be material for realizing the present invention in diverse forms thereof.

## Claims

1. A retrieval basket (112) for entrapping a foreign object located in a body organ or passage, comprising
a first section (122), suitable for capturing the object; and
a second section (124), suitable for retention the captured object;
where said first section (122) includes at least two branches, distal ends of at least some of the branches are provided with loops (134) emerging therefrom, and said loops (134) spatially define the second section (122) of the basket (112),
**characterized in that**
at least some of the distal ends of the loops (134) are intertwined one with another so as to define collectively a net having a cell structure capable to retain the captured object within the second section (124) of the basket (112).

2. The basket (112) as defined in claim 1, in which the branches are made of wire filaments.

3. The basket (112) as defined in claim 2, in which said loops (134) are configured from said wire filaments.

4. The basket (112) as defined in claims 1, in which the loops (134) are made of separate wire filaments and connected to the branches.

5. The basket (112) as defined in claims 4, in which the loops (134) are connected to the branches by soldering.

6. The basket (112) as defined in claim 2 or 3, in which said wire filaments of the branches are made of metallic material.

7. The basket (112) as defined in claim 4 or 5, in which said separate wire filaments of the loops are made of metallic material.

8. The basket (112) as defined in claim 6 or 7, in which said metallic material has shape memory characteristic.

9. The basket (112) as defined in claim 8, in which said metallic material is Ni-Ti based alloy.

10. The basket (112) as defined in claim 6 or 7, in which said metallic material is stainless steel.

11. The basket (112) as defined in claims 2 or 3, in which said wire filaments of the branches are made of organic material.

12. The basket (112) as defined in claim 4 or 5, in which said separate wire filaments of the loops (134) are made of organic material.

13. The basket (112) as defined in claim 11 or 12, in which said organic material is Capron.

14. The basket (112) as defined in any one of claims 2, 3, 6 and 11, in which said wire filaments of the branches are coated with a coating layer.

15. The basket (112) as defined in any one of claims 4, 7 and 12, in which said separate wire filaments of the loops (134) are coated with a coating layer.

16. The basket (112) as defined in claim 14 or 15, in which said coating layer is Teflon.

17. The basket (112) as defined in any one of claims 2, 3, 6 and 11, in which a diameter of said wire filaments is in the range of about 0.1 to 0.4mm.

18. The basket (112) as defined in claim 14 or 15, in which a diameter of said separate wire filaments is in the range of about 0.1 to 0.4mm.

19. The basket (112) as defined in any one of preceding claims, in which said foreign object is selected from the group including stones from urethra, gall stones and calculi.

20. The basket (112) as defined in any one of preceding claims, in which at least some of said loops are configured in an 8-like fashion.

21. The basket (112) as defined in any one of preceding claims, in which size of cells of said net is decreased towards the distal end of the basket (112).

22. A surgical device (100) for evacuation of said foreign object from the body organ or passage comprising a retrieval basket (112) as claimed in claim 1; and a basket control means (114) coupled to said retrieval basket (112), said basket control means (114) comprises:
a sheath (116) insertable within the body organ for reaching the object; and
a manipulator (120) coupled to the basket (112) via a pushing rod (118) or cable or wire arranged inside said sheath (116), where said manipulator (120) is suitable either for retracting the basket (112) within the sheath (116) or for protracting the basket (112) from the sheath (116) to enable its opening.

23. The surgical device (100) of claim 22, in which said basket control means (114) further comprises a power source electrically connected to the basket (112).

## Patentansprüche

1. Extraktionskörbchen (112) zum Einfangen eines Fremdkörpers, der sich in einem Körperorgan oder -Durchgang befindet, umfassend
einen ersten Abschnitt (122), der zum Einfangen des Körpers geeignet ist; und
einen zweiten Abschnitt (124), der zum Festhalten des eingefangenen Körpers geeignet ist;
wobei der erste Abschnitt (122) mindestens zwei Zweige einschließt, die distalen Enden mindestens einiger der Zweige mit daraus austretenden Schlingen (134) versehen sind, und die Schlingen (134) den zweiten Abschnitt (122) des Körbchens (112) räumlich begrenzen,
**dadurch gekennzeichnet, dass**
mindestens einige der distalen Enden der Schlingen (134) miteinander verschlungen sind, um so gemeinsam ein Netz mit einer Zellenstruktur zu bilden, die den eingefangenen Körper innerhalb des zweiten Abschnitts (124) des Körbchens (112) festhalten kann.

2. Körbchen (112) nach Anspruch 1, bei dem die Zweige aus Drahtfäden bestehen.

3. Körbchen (112) nach Anspruch 2, bei dem die Schlingen (134) aus den Drahtfäden konfiguriert sind.

4. Körbchen (112) nach Anspruch 1, bei dem die Schlingen (134) aus getrennten Drahtfäden bestehen und mit den Zweigen verbunden sind.

5. Körbchen (112) nach Anspruch 4, bei dem die Schlingen (134) durch Löten mit den Zweigen verbunden werden.

6. Körbchen (112) nach Anspruch 2 oder 3, bei dem die Drahtfäden der Zweige aus Metallmaterial bestehen.

7. Körbchen (112) nach Anspruch 4 oder 5, bei dem die getrennten Drahtfäden der Schlingen aus Metallmaterial bestehen.

8. Körbchen (112) nach Anspruch 6 oder 7, bei dem das Metallmaterial eine Formgedächtnischarakteristik aufweist.

9. Körbchen (112) nach Anspruch 8, bei dem das Metallmaterial eine auf Ni-Ti basierende Legierung ist.

10. Körbchen (112) nach Anspruch 6 oder 7, bei dem das Metallmaterial Edelstahl ist.

11. Körbchen (112) nach Anspruch 2 oder 3, bei dem die Drahtfäden der Zweige aus organischem Material bestehen.

12. Körbchen (112) nach Anspruch 4 oder 5, bei dem die getrennten Drahtfäden der Schlingen (134) aus organischem Material bestehen.

13. Körbchen (112) nach Anspruch 11 oder 12, bei dem das organische Material Capron darstellt.

14. Körbchen (112) nach einem der Ansprüche 2, 3, 6 und 11, bei dem die Drahtfäden der Zweige mit einer Deckschicht überzogen sind.

15. Körbchen (112) nach einem der Ansprüche 4, 7 und 12, bei dem die getrennten Drahtfäden der Schlingen (134) mit einer Deckschicht überzogen sind.

16. Körbchen (112) nach Anspruch 14 oder 15, bei dem die Deckschicht Teflon darstellt.

17. Körbchen (112) nach einem der Ansprüche 2, 3, 6 und 11, bei dem ein Durchmesser der Drahtfäden im Bereich von etwa 0,1 bis 0,4 mm liegt.

18. Körbchen (112) nach Anspruch 14 oder 15, bei dem ein Durchmesser der getrennten Drahtfäden im Bereich von etwa 0,1 bis 0,4 mm liegt.

19. Körbchen (112) nach einem der vorhergehenden Ansprüche, bei dem der Fremdkörper aus der Gruppe ausgewählt ist, die Steine aus der Harnröhre, Gallensteine und Nierensteine einschließt.

20. Körbchen (112) nach einem der vorhergehenden Ansprüche, bei dem mindestens einige der Schlingen in einer 8-artigen Weise konfiguriert sind.

21. Körbchen (112) nach einem der vorhergehenden Ansprüche, bei dem die Größe von Zellen des Netzes in Richtung des distalen Endes des Körbchens (112) verkleinert wird.

22. Chirurgische Einrichtung (100) zur Evakuierung des Fremdkörpers aus dem Körperorgan oder -Durchgang, die ein Extraktionskörbchen (112) nach Anspruch 1; und ein Körbchensteuermittel (114) gekoppelt an das Extraktionskörbchen (112) aufweist, wobei das Körbchensteuermittel (114) Folgendes aufweist:
eine Scheide (116), die in das Körperorgan zum Erreichen des Körpers einführbar ist; und einen Manipulator (120), der an das Körbchen (112) über eine Schubstange (120) oder ein Kabel oder einen Draht angeordnet innerhalb der Scheide (116) gekoppelt ist, wobei der Manipulator (120) entweder zum Zurückziehen des Körbchens (112) in die Scheide (116) oder zum Vorschieben des Körbchens (112) aus der Scheide (116) zum Ermöglichen seiner Öffnung geeignet ist.

23. Chirurgische Einrichtung (100) nach Anspruch 22, bei der das Körbchensteuermittel (114) weiter eine Stromquelle aufweist, die elektrisch an das Körbchen (112) gekoppelt ist.

## Revendications

1. Panier d'extraction (112) servant à attraper un corps étranger se trouvant dans un organe ou conduit du corps humain, comportant
une première section (122), adaptée pour capter le corps étranger ; et
une deuxième section (124), adaptée pour retenir le corps étranger capté ;
dans lequel ladite première section (122) comprend au moins deux branches, les extrémités distales d'au moins quelques-unes des branches étant mises en oeuvre avec des boucles (134) émergeant en provenance de celles-ci, et dans lequel lesdites boucles (134) définissent de manière spatiale la deuxième section (122) du panier (112),
**caractérisé en ce que**
au moins quelques-unes des extrémités distales des boucles (134) sont entrelacées les unes avec les autres de manière à définir collectivement un filet ayant une structure en forme d'alvéole capable de retenir le corps étranger capté à l'intérieur de la deuxième section (124) du panier (112).

2. Panier (112) selon la revendication 1, dans lequel les branches sont réalisées à partir de filaments métalliques.

3. Panier (112) selon la revendication 2, dans lequel lesdites boucles (134) sont configurées à partir desdits filaments métalliques.

4. Panier (112) selon la revendication 1, dans lequel les boucles (134) sont réalisées à partir de filaments métalliques séparés et sont raccordées sur les branches.

5. Panier (112) selon la revendication 4, dans lequel les boucles (134) sont raccordées sur les branches par soudage.

6. Panier (112) selon la revendication 2 ou la revendication 3, dans lequel lesdits filaments métalliques des branches sont réalisés à partir d'un matériau métallique.

7. Panier (112) selon la revendication 4 ou la revendication 5, dans lequel lesdits filaments métalliques séparés des boucles sont réalisés à partir d'un matériau métallique.

8. Panier (112) selon la revendication 6 ou la revendication 7, dans lequel ledit matériau métallique a une caractéristique de mémoire de forme.

9. Panier (112) selon la revendication 8, dans lequel ledit matériau métallique est un alliage à base de Ni-Ti.

10. Panier (112) selon la revendication 6 ou la revendication 7, dans lequel ledit matériau métallique est de l'acier inoxydable.

11. Panier (1 12) selon la revendication 2 ou la revendication 3, dans lequel lesdits filaments métalliques des branches sont réalisés à partir d'un matériau organique.

12. Panier (112) selon la revendication 4 ou la revendication 5, dans lequel lesdits filaments métalliques séparés des boucles (134) sont réalisés à partir d'un matériau organique.

13. Panier (112) selon la revendication 11 ou la revendication 12, dans lequel ledit matériau organique est du Capron.

14. Panier (112) selon l'une quelconque des revendications 2, 3, 6 et 11, dans lequel lesdits filaments métalliques des branches sont revêtus d'une couche de revêtement.

15. Panier (112) selon l'une quelconque des revendications 4, 7 et 12, dans lequel lesdits filaments métalliques séparés des boucles (134) sont revêtus d'une couche de revêtement.

16. Panier (112) selon la revendication 14 ou la revendication 15, dans lequel ladite couche de revêtement est du Téflon.

17. Panier (112) selon l'une quelconque des revendications 2, 3, 6 et 11, dans lequel un diamètre desdits filaments métalliques est de l'ordre d'environ 0,1 à 0,4 mm.

18. Panier (112) selon la revendication 14 ou la revendication 15, dans lequel un diamètre desdits filaments métalliques séparés est de l'ordre d'environ 0,1 à 0,4 mm.

19. Panier (112) selon l'une quelconque des revendications précédentes, dans lequel ledit corps étranger est sélectionné dans le groupe comprenant des calculs en provenance de l'urètre, des calculs biliaires et des calculs.

20. Panier (112) selon l'une quelconque des revendications précédentes, dans lequel au moins quelques-unes desdites boucles sont configurées en une forme de 8.

21. Panier (112) selon l'une quelconque des revendications précédentes, dans lequel la taille des alvéoles dudit filet va en diminuant vers l'extrémité distale du panier (112).

22. Dispositif chirurgical (100) à des fins d'évacuation dudit corps étranger en provenance de l'organe ou conduit du corps humain comportant un panier d'extraction (112) selon la revendication 1 ; et un moyen de commande du panier (114) couplé sur ledit panier d'extraction (112), ledit moyen de commande du panier (114) comportant :
un manchon (116) insérable à l'intérieur de l'organe du corps humain pour atteindre le corps étranger ; et
un manipulateur (120) couplé au panier (112) par le biais d'une tige de poussée (118) ou d'un câble ou d'un fil arrangé à l'intérieur dudit manchon (116), ledit manipulateur (120) étant adapté soit pour faire rentrer le panier (112) à l'intérieur du manchon (116) soit pour faire sortir le panier (112) du manchon (116) pour permettre son ouverture.

23. Dispositif chirurgical (100) selon la revendication 22, dans lequel ledit moyen de commande du panier (114) comporte par ailleurs une source d'alimentation connectée de manière électrique sur le panier (112).
